# EUROPEAN PATENT APPLICATION

(11) **EP 4 821 431 A1**
(43) Date of publication of application: **14.10.2026**
(21) Application number: 25169773.6
(22) Date of filing: 10.04.2025
(51) Int. Cl.: A61B 5/08

(54) **DEVICE AND METHOD FOR MEASURING GAS IN PERSONAL CARE DEVICES**

(71) Applicant: Koninklijke Philips N.V., 5656 AE Eindhoven (NL)
(72) Inventor: SJOBERG, Kurt, Eindhoven (NL); VAN DEN DUNGEN, Wilhelmus Andreas Marinus Arnoldus Maria, Eindhoven (NL); KOOIJMAN, Gerben, Eindhoven (NL)
(74) Representative: Philips Intellectual Property & Standards

(57) **Abstract**

The described technology pertains to personal care devices equipped with gas measurement technologies for monitoring oral-systemic health. Technology addresses the challenge of accurately measuring gas compounds in exhaled breath, which can be confounded by ambient air gases. The solution involves a device (100) with a first sensor (101) for gas measurement, a proximity detection means (102), and a processor (103) that controls background and exhaled gas measurements. The processor processes these measurements to provide a gas output signal representing user-produced gases. This system enhances health assessments by distinguishing between ambient and user-produced gases, offering non-invasive, real-time monitoring. The device is particularly useful in personal care applications, such as toothbrushes, where consistent monitoring is beneficial.

## Description

### FIELD OF THE INVENTION

The present disclosure relates to personal care devices, specifically those incorporating gas measurement technologies for monitoring oral-systemic health by detecting gas compounds in exhaled breath.

### BACKGROUND OF THE INVENTION

In recent years, there has been a growing recognition of the connection between oral health and overall systemic health. Monitoring gases in exhaled breath has emerged as a promising method for detecting early signs of systemic diseases, offering a non-invasive approach to health assessment. This method is particularly advantageous when integrated into tools used regularly, which can provide consistent monitoring. However, accurately measuring gas compounds in exhaled breath presents challenges, as certain compounds of interest may also be present in the ambient air, potentially leading to inaccurate assessments.

Existing solutions often struggle with distinguishing between gases produced by the user and those present in the surrounding environment. This is further complicated by the cross-sensitivity of sensors, which can respond to non-target compounds, affecting the reliability of the measurements. These limitations highlight the need for improved methods and devices that can effectively differentiate between ambient and user-produced gases, ensuring accurate and reliable health monitoring through breath analysis.

### SUMMARY OF THE INVENTION

In a first aspect of the invention, a personal care device (100) is presented, comprising: a first sensor (101) for measuring gas compounds; a means (102) for detecting proximity of a user to the personal care device, the means providing a proximity output signal; a processor (103) configured for: controlling the first sensor (101) for performing a background gas measurement, in response to the proximity output signal; thereafter controlling the first sensor (101) for performing an exhaled gas measurement; processing the background gas measurement and the exhaled gas measurement to provide a gas output signal representing gas produced by the user.

According to embodiments, the means (102) is a touch-sensitive proximity sensor.

According to embodiments, the means (102) is an IMU.

According to embodiments, the processor (103) is further configured to control the first sensor (101) such that the background gas measurement is stopped when the personal care device (100) is switched on.

According to embodiments, the processor (103) is further configured to control the first sensor (101) such that the background gas measurement is stopped when fluctuations in the first sensor (101) output signal are observed.

According to embodiments, the personal care device (100) further comprises a second sensor (104) for detecting proximity of the personal care device (100) to a user's face, and wherein the processor (103) is further configured to control the first sensor (101) such that the background gas measurement is stopped when the personal care device (100) is in proximity of the user's face within a predetermined limit.

According to embodiments, processing the background gas measurement and the exhaled gas measurement to provide a gas output signal comprises discarding or selecting a portion of the background gas measurement.

According to embodiments, the background gas measurement is performed when the proximity signal indicates that the user is in proximity of the device (100).

According to embodiments, the background gas measurement is performed as long as the proximity signal indicates that the user is not in proximity of the device.

According to embodiments, the timing of performing the background gas measurement is determined by using historic use data of the personal care device (100).

In a second aspect of the invention, a method (200) for providing a gas output signal representing gas produced by a user using a personal care device (100) is presented, comprising: sensing (201) proximity of a user to the personal care device (100); controlling (202) a background gas measurement in response to the sensed user proximity signal; measuring (203) exhaled gas of the user resulting in a exhaled gas measurement; processing (204) the background gas measurement and the exhaled gas measurement to provide a gas output signal representing gas produced by the user.

According to embodiments, the background gas measurement is stopped (205) when the user is in proximity of the personal care device (100).

According to embodiments, the background gas measurement is stopped when fluctuations in the background gas measurement signal are observed (206).

According to embodiments, the method further comprises detecting proximity of the personal care device (100) to a user's face, and wherein the background gas measurement is stopped when the personal care device (100) is in proximity from the user's face (207).

According to embodiments, the background gas measurement is performed when the user proximity signal indicates that the user is in proximity of the device.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### BRIEF DESCRIPTION OF THE DRAWINGS

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.
FIG. 1 Illustrates a personal care device (100) comprising a first sensor (101) for measuring gas compounds, a means (102) for detecting proximity of a user to the device, and a processor (103) for processing the measurements.
FIG. 2 Shows a personal care device (100) similar to FIG. 1 but additionally includes a second sensor (104) for detecting proximity of the device to a user's face.
FIG. 3 Depicts a flowchart of a method (200) for providing a gas output signal, including steps of sensing proximity (201), performing a background gas measurement (202), measuring exhaled gas (203), and processing the measurements (204).
FIG. 4 Illustrates a flowchart similar to FIG. 3, with an additional step (205) to stop the background gas measurement when the personal care device is switched on.
FIG. 5 Shows a flowchart similar to FIG. 3, with an additional step (206) to stop the background gas measurement when fluctuations in the background gas measurement signal are observed.
FIG. 6 Depicts a flowchart similar to FIG. 3, with an additional step (207) for detecting proximity of the device to a user's face and stopping the background gas measurement when the device is near the user's face.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some, but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In recent years, the connection between oral health and overall systemic health has gained significant attention. Monitoring gases in exhaled breath has emerged as a promising method for detecting early signs of systemic diseases, offering a non-invasive approach to health assessment. This method is particularly advantageous when integrated into tools used regularly, such as personal care devices, which can provide consistent monitoring. However, accurately measuring gas compounds in exhaled breath presents challenges, as certain compounds of interest may also be present in the ambient air, potentially leading to inaccurate assessments.

The presented invention in this disclosure addresses these challenges of the state of the art described in the background section by providing a personal care device equipped with advanced gas measurement capabilities. The device includes a sensor for measuring target gas compounds in the user's breath and/or oral cavity, along with means to perform a background measurement of gas compounds in the ambient air. This enables the device to distinguish between compounds or compound concentrations already present in the environment and those produced by the user. By performing background measurements close in time to the use of the device, the technology ensures that fluctuations in gas compound concentrations and sensor response drift are accounted for, thereby enhancing the accuracy of the health assessment.

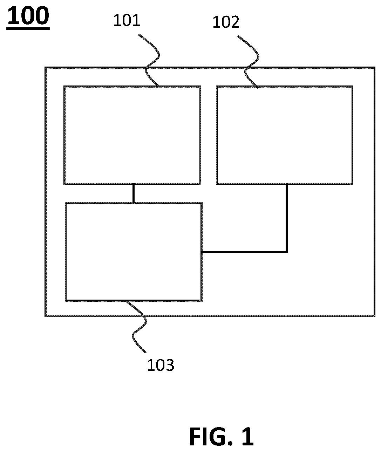

An embodiment of the **first** aspect of the invention is illustrated in FIG. 1. FIG. 1 illustrates a personal care device 100, which includes a first sensor 101, a means for detecting proximity of a user to the personal care device 102, and a processor 103. This personal care device 100 is designed to measure gas compounds and process these measurements to provide a gas output signal representing gas produced by the user. The first sensor 101 is responsible for measuring gas compounds. This sensor is configured to detect specific gases, for example in the user's exhaled breath or user's air sent out of the lungs, which are indicative of various health conditions. The first sensor 101 is sensitive to the target gas compounds and is capable of distinguishing these from other gases present in the environment. The means for detecting proximity of a user to the personal care device 102 is a component that detects when a user is near the personal care device 100. This component provides a proximity output signal that triggers the operation of the first sensor 101. The processor 103 is configured to perform several functions. It initiates a background gas measurement using the first sensor 101 in response to the proximity output signal from the means for detecting proximity of a user to the personal care device 102. Afterward, the processor is configured to measure the exhaled gas of the user using the first sensor 101 and processes both the background and exhaled gas measurements to generate a gas output signal. This gas output signal represents the gas produced by the user, allowing for an accurate assessment of the user's health based on the detected gas compounds.

The integration of a first sensor, a proximity detection means, and a processor within the personal care device 100 creates a sophisticated system capable of accurately measuring and analyzing gas compounds in a user's exhaled breath. The first sensor 101 is specifically designed to detect target gas compounds that are indicative of various health conditions, such as volatile organic compounds (VOCs) that may signal metabolic or respiratory issues.

The proximity detection means 102 plays a crucial role by providing a proximity output signal when a user is near the device. This signal triggers the first sensor 101 to start or stop a background gas measurement, which is essential for establishing a baseline of ambient gas concentrations. By doing so, the device can differentiate between gases present in the environment and those exhaled by the user. This differentiation is critical for accurate health assessments, as it ensures that the gas output signal generated by the processor 103 truly reflects the user's exhaled gases rather than ambient interference. The processor 103 is the central component that controls the device's operations. It controls the first sensor 101 to perform background gas measurements in response to the proximity signal, further controls the first sensor 101 to measure exhaled gases, and processes both sets of data to produce a gas output signal. This signal represents the gas produced by the user, providing a reliable basis for assessing the user's health. The processor's ability to handle these tasks efficiently ensures that the device operates seamlessly, delivering accurate and timely health insights.

The advantage of this integrated system is its ability to provide non-invasive, real-time health monitoring through breath analysis. By accurately distinguishing between ambient and user-produced gases, the device offers precise health assessments that can be used to detect early signs of systemic diseases. This capability is particularly valuable in personal care devices, such as toothbrushes, which are used regularly and can provide consistent monitoring without requiring additional effort from the user. The use of a proximity detection means enhances user convenience and device efficiency. By activating the first sensor 101, for example only when the user is near, the device conserves power and extends the lifespan of its components. This feature also ensures that measurements are taken at optimal times, reducing the likelihood of ambient interference and improving the reliability of the health assessments. Similarly, by activating the first sensor 101, for example only when the user is not near, a more accurate background gas measurement can be performed as the surrounding air is not contaminated by any components introduced by the user's presence.

Overall, the integration of these components within the personal care device 100 results in a user-friendly, efficient, and accurate system for health monitoring. It empowers users to gain insights into their health status through a routine activity, such as brushing their teeth, making it a practical and valuable tool for proactive health management.

Throughout the description it is described that an exhaled gas measurement is performed by the first sensor. Controlling the first sensor for performing an exhaled gas measurement involves timing the activation of the sensor to capture the user's exhaled gases accurately. The timing of such measurement may be, for example: 1) during an oral care routine: Activate the sensor when the user begins an oral care routine, such as brushing teeth. For example, when the user switches on the device. The processor may be configured to control the sensor in such a manner. This timing ensures that the sensor captures exhaled gases when the user's mouth is open and actively engaged in the routine, providing a clear opportunity for gas measurement; 2) after the background gas measurement: Initiate the exhaled gas measurement immediately following a background gas measurement. The processor may be configured to control the sensor in such a manner. This sequence allows the device to establish a baseline of ambient gas concentrations first, ensuring that subsequent exhaled gas measurements are corrected for ambient interference; 3) upon proximity detection: trigger the sensor when the proximity detection means indicates that the user is near the device, such as when the device is brought close to the face or mouth. The processor may be configured to control the sensor in such a manner. This ensures that the measurement occurs when the user is likely to be exhaling near the device; 4) a user-activated timing: allow the user to manually activate the sensor for exhaled gas measurement, such as pressing a button or using a voice command. The processor may be configured to control the sensor in such a manner. This user-driven timing provides flexibility and ensures that the measurement is taken at a convenient moment for the user; 5) a scheduled measurement: implement scheduled timing based on historical usage patterns, where the device automatically performs exhaled gas measurements at times when the user typically engages in oral care activities. The processor may be configured to control the sensor in such a manner. This predictive timing leverages past data to optimize measurement opportunities. By strategically timing the activation of the first sensor for exhaled gas measurement, the device can ensure accurate and reliable data collection, leading to more precise health assessments.

According to an embodiment, the personal care device is a toothbrush or a hair treatment device such as a shaver, clipper or trimmer.

According to an embodiment of the invention, the means for detecting proximity of a user to the personal care device 102 is a touch-sensitive proximity sensor. According to embodiments, the touch-sensitive proximity sensor may be an optical, a capacitive or a mechanical-based sensor (e.g. a switch or push button). The optical sensor may be an infrared or time-of-flight sensor, detecting proximity by measuring changes in light reflection or travel time. As an advantage, the optical sensor can accurately sense the presence and distance of objects, including a user's hand or face, without requiring physical contact. Capacitive sensors may detect changes in capacitance caused by the presence of conductive materials, such as the human body. They can sense proximity by measuring the electrical field disturbance when a user approaches the device. Mechanical-based sensors, such as switches or push buttons, detect proximity through physical interaction. They require the user to press or activate the sensor, providing a clear indication of user presence.

This sensor generates a proximity output signal when a user is close to, approaches or touches the device, triggering the initiation or the stop of background gas measurements. This sensor integration allows for seamless operation, ensuring that background gas measurements are performed at optimal times, thereby improving the accuracy of distinguishing between ambient and user-produced gases. This leads to more reliable health assessments and enhances the overall user experience by providing consistent and accurate monitoring of exhaled gases.

According to an embodiment of the invention, the means for detecting proximity of a user to the personal care device 102 is an IMU.

An IMU detects motion and orientation changes by measuring specific forces, angular rates, and sometimes magnetic fields. When integrated into a personal care device, the IMU can sense when the device is picked up, moved, or oriented in a particular way that indicates user interaction. This capability allows the IMU to generate a signal indicating the proximity or handling of the device by the user, which can be used to trigger background gas measurements. The advantage of using an IMU is its ability to detect dynamic user interactions with the device, such as picking up or moving the device, without requiring direct contact or line-of-sight. This capability enhances the device's responsiveness to user actions, ensuring that background gas measurements are initiated precisely when the device is likely to be used. Additionally, IMUs are compact and can be easily integrated into the design of personal care devices, providing robust motion detection without adding significant weight or complexity. This integration allows for more accurate timing of gas measurements, improving the distinction between ambient and user-produced gases, leading to more reliable health assessments. Furthermore, the use of an IMU can contribute to a more intuitive and seamless user experience, as the device can automatically respond to natural user movements.

According to an embodiment of the invention, processor 103 is further configured to control the first sensor (101) such that the background gas measurement is stopped when the personal care device 100 is switched on. In a further embodiment, the processor 103 is further configured to control the first sensor (101) such that the exhaled gas measurement is started when the personal care device 100 is switched on.

Configuring the processor to control the first sensor 101 to stop the background gas measurement when the personal care device is switched on or wakes up from a standby state, ensures that the device transitions from measuring ambient gases to focusing on user-produced gases. This configuration prevents the overlap of background and exhaled gas measurements, thereby maintaining the integrity and accuracy of the data collected for health assessments. The advantage of this configuration is the enhancement of measurement accuracy. By stopping the background gas measurement when the device is switched on or wakes up, the system ensures that only relevant user-produced gas data is captured during active use. This reduces the risk of ambient gas interference, leading to more precise health assessments. This contributes to a more reliable and user-friendly experience, as the device provides consistent and accurate monitoring of exhaled gases without manual intervention.

According to an embodiment, the processor 103 is further configured to control the first sensor 101 such that the background gas measurement is stopped when fluctuations in the first sensor 101 output signal are observed.

Configuring the processor to control the first sensor 101 to stop the background gas measurement when fluctuations in the first sensor's output signal are observed allows the device to dynamically respond to changes in the measurement environment. This capability ensures that the device can distinguish between stable ambient conditions and those that may indicate the presence of user-produced gases, thereby optimizing the timing of gas measurements. The advantage of this configuration is the improvement in measurement accuracy and reliability. By halting background gas measurements in response to fluctuations, the device minimizes the risk of capturing erroneous data that could result from transient environmental changes or sensor noise. This leads to more accurate assessments of user-produced gases, enhancing the overall reliability of health monitoring. Additionally, this approach conserves sensor resources and processing power by avoiding unnecessary background measurements during periods of instability, contributing to the device's operational efficiency and extending its battery life. This dynamic response capability also enhances the user experience by ensuring that the device adapts to varying conditions without requiring manual adjustments.

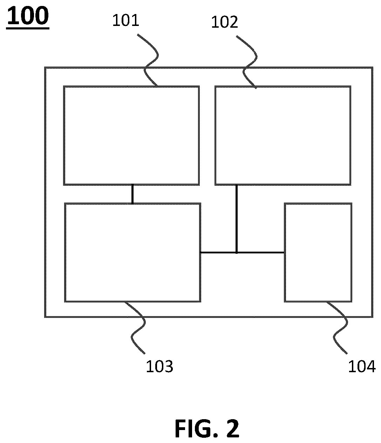

According to an embodiment of the invention and illustrated in FIG. 2, the personal care device 100 further comprises a second sensor 104 for detecting proximity of the personal care device 100 to a user's face, and wherein the processor 103 is further configured to control the first sensor 101 such that the background gas measurement is stopped when the personal care device 100 is in proximity of the user's face, within a predetermined limit. Such predetermined limit can be between 0-50 cms, being the distance range between the personal care device 100 and the user's face. In the preferred embodiment, the range may be 0-20 cms.

The inclusion of a second sensor 104 for detecting the proximity of the personal care device to a user's face enhances the device's ability to accurately determine when to transition from background gas measurement to user-produced gas measurement. This second sensor 104 provides an additional layer of proximity detection, specifically focusing on the device's closeness to the user's face. When the second sensor 104 detects that the device is near the user's face, it signals the processor 103 to stop the background gas measurement. This ensures that the device is ready to measure exhaled gases at the precise moment when the user is likely to be exhaling near the device, thereby improving the accuracy of the gas output signal. This configuration improves user experience by automating the transition between background and exhaled gas measurements, reducing the need for manual intervention or adjustments. The device becomes more intuitive and responsive to natural user interactions, such as bringing the device close to the face during use. This seamless operation contributes to a more user-friendly and efficient personal care device, encouraging regular use and consistent health tracking.

According to an embodiment, processing the background gas measurement and the exhaled gas measurement to provide a gas output signal comprises discarding a portion of the background gas measurement. This approach effectively eliminates any residual ambient gas data that might otherwise skew the results, particularly when the device transitions from measuring ambient air to measuring exhaled breath. By discarding irrelevant background data, the system refines the accuracy of the gas output signal, focusing solely on the user-produced gases. The primary advantage of this method is the enhancement of measurement accuracy and reliability. By discarding portions of the background gas measurement that are no longer relevant, the system ensures that the gas output signal is based solely on user-produced gases, leading to more precise health assessments. Additionally, this method simplifies the data processing requirements, allowing the system to focus on analyzing the most relevant data. This improves the efficiency of the device, providing quicker and more accurate results while conserving processing power. The streamlined operation not only enhances the user experience but also extends the device's operational lifespan by reducing unnecessary data handling.

In a particular embodiment, processing the background gas measurement and the exhaled gas measurement to provide a gas output signal comprises discarding a portion of the background gas measurement when it is detected that the personal care device 100 is in the mouth of the user.

By processing the background gas measurement and the exhaled gas measurement to provide a gas output signal and specifically discarding a portion of the background gas measurement when it is detected that the personal care device is in the mouth of the user, the system ensures that the gas output signal accurately reflects the gases produced by the user. This approach effectively eliminates any residual ambient gas data that might otherwise skew the results, particularly when the device transitions from measuring ambient air to measuring exhaled breath. The detection of the device being in the user's mouth serves as a trigger to discard irrelevant background data, thereby refining the accuracy of the gas output signal. The advantage of this method is the enhancement of measurement accuracy and reliability. By discarding portions of the background gas measurement that are no longer relevant once the device is in the user's mouth, the system ensures that the gas output signal is based solely on user-produced gases. This leads to more precise health assessments, as the measurements are not contaminated by ambient air data that could otherwise lead to false readings. Additionally, this approach simplifies the data processing requirements, as the system can focus on analyzing the most relevant data, thereby improving the efficiency of the device. This efficiency not only enhances the user experience by providing quicker and more accurate results but also conserves processing power and extends the device's operational lifespan. Overall, this method contributes to a more reliable and user-friendly personal care device, encouraging regular use and consistent health monitoring.

To facilitate detection of the personal care device 100 in the mouth, an additional means for detecting insertion in the mouth may be present in the personal care device. This may be a pressure sensor, a moisture sensor, an optical sensor, a sound sensor, a conductivity sensor. Also, the means 102 for detecting proximity of a user to the personal care device may be configured to detect insertion in the mouth. For example, when the means 102 is an IMU whereby from the IMU data the processor 103 may determine when the device 100 is inserted into the mouth.

Discarding a portion of the background gas measurement may be realized using the following process:
**Sensor Calibration:** Initially, the sensor must be calibrated to establish baseline readings for ambient gas concentrations. This calibration helps in identifying the typical range of background gas levels when the device is not in use.
**Data Segmentation:** During operation, the sensor continuously collects data on gas concentrations. This data is segmented into background and exhaled gas measurements based on the timing and proximity signals from the device.
**Threshold Setting:** Establish thresholds for distinguishing between ambient and user-produced gases. These thresholds are based on the sensor's calibration and historical data, allowing the system to identify when the device transitions from measuring background gases to exhaled gases.
**Proximity Detection:** Utilize proximity sensors to detect when the device is in use or near the user's mouth. This detection triggers the transition from background measurement to exhaled gas measurement.
**Data Filtering:** Implement algorithms to filter out portions of the background gas measurement that fall outside the established thresholds or occur during the proximity detection phase. This filtering process discards irrelevant data that does not contribute to the accurate assessment of user-produced gases.
**Signal Processing:** Use signal processing techniques to analyze the filtered data, ensuring that the gas output signal accurately represents the user's exhaled gases. This involves comparing the filtered background data with the exhaled gas data to refine the output signal.
**Machine Learning Integration:** Optionally, integrate machine learning models to continuously improve the accuracy of data segmentation and filtering. These models can learn from historical data to better predict and discard irrelevant background measurements.

According to an embodiment, processing the background gas measurement and the exhaled gas measurement to provide a gas output signal comprises selecting a portion of the background gas measurement.

Processing the background gas measurement and the exhaled gas measurement to provide a gas output signal by selecting a portion of the background gas measurement allows the system to refine the accuracy of the gas output signal. By carefully selecting relevant portions of the background data, the system can establish a precise baseline of ambient gas concentrations. The advantage of this method is the enhancement of measurement precision and reliability. By selecting only the most relevant portions of the background gas measurement, the system can more accurately correct the exhaled gas data for ambient interference. This leads to more precise health assessments, as the gas output signal is less likely to be influenced by irrelevant ambient conditions.

Additionally, this approach optimizes the device's data processing efficiency by focusing on the most pertinent background data, reducing the computational load and conserving processing power. This targeted data selection contributes to quicker and more accurate results, enhancing the overall user experience. The streamlined operation not only improves the reliability of health monitoring but also extends the device's operational lifespan by minimizing unnecessary data handling. Overall, this method supports a more user-friendly and effective personal care device, encouraging regular use and consistent health tracking.

According to an embodiment, the background gas measurement is performed when the proximity signal indicates that the user is in proximity of the device 100, e.g. withing a predetermined limit. Such predetermined limit can be between 0-100 cms, being the distance range between the personal care device 100 and the user. In the preferred embodiment, the range may be 0-50 cms.

Performing the background gas measurement when the proximity signal indicates that the user is in proximity of the device ensures that the measurement is conducted under conditions that closely resemble the environment in which the exhaled gas measurement will occur. This approach allows the device to establish an accurate baseline of ambient gas concentrations just before the user begins using the device. By aligning the timing of the background measurement with the user's proximity, the device can account for any immediate changes in the ambient environment, such as those caused by the user's presence or movement. The advantage of this approach is the enhancement of measurement accuracy and reliability. Additionally, this method optimizes the device's operational efficiency by ensuring that background measurements are only taken when necessary, conserving sensor resources and processing power. This targeted approach reduces the likelihood of unnecessary measurements, extending the device's battery life and improving the overall user experience. By providing accurate and timely health insights, the device encourages regular use and consistent health monitoring, making it a valuable tool for proactive health management.

According to an embodiment, the background gas measurement is performed as long as the proximity signal indicates that the user is not in proximity of the device 100, e.g. within a predetermined limit. Such predetermined limit can be between 0-100 cms, being the distance range between the personal care device 100 and the user. In the preferred embodiment, the range may be 0-50 cms.

Performing the background gas measurement when the proximity signal indicates that the user is not in proximity of the device allows the system to establish a baseline of ambient gas concentrations without the influence of user-produced gases. This approach ensures that the background measurement reflects the true ambient conditions, free from any immediate impact of the user's presence or exhaled gases. By capturing the ambient gas levels when the user is not near the device, the system can more accurately differentiate between ambient and user-produced gases during subsequent measurements. The advantage of this approach is the enhancement of the accuracy and reliability of health assessments. By conducting background gas measurements when the user is not in proximity, the device can ensure that the baseline data is not contaminated by user-produced gases. This leads to more precise differentiation between ambient and exhaled gases, resulting in more accurate health assessments. Additionally, this method allows the device to perform background measurements at times when it is not actively being used, optimizing the use of sensor resources and processing power. This can contribute to extending the device's battery life and reducing wear on the sensors, enhancing the overall efficiency and longevity of the device. By ensuring that background measurements are conducted under optimal conditions, the device can provide more reliable health insights, encouraging regular use and consistent health monitoring.

According to an embodiment, the timing of performing the background gas measurement is determined using historic data of the personal care device 100.

When to perform a background gas measurement maybe done using historic use data of the personal care device allowing the system to leverage past usage patterns to predict and optimize the timing of background measurements. By analyzing historical data, the device can identify typical environmental conditions and user behaviors, enabling it to anticipate when background gas measurements should be conducted. This predictive capability ensures that the device can establish an accurate baseline of ambient gas concentrations that is tailored to the user's specific usage patterns. The advantage of using historic use data for background gas measurements is the enhancement of measurement accuracy and personalization. By tailoring the timing of background measurements to the user's typical usage patterns, the device can more effectively account for variations in ambient conditions that are specific to the user's environment. This leads to more precise differentiation between ambient and user-produced gases, resulting in more accurate health assessments. Additionally, this approach optimizes the device's operational efficiency by reducing the need for unnecessary measurements. By predicting when background measurements are most needed, the device can conserve sensor resources and processing power, extending battery life and reducing sensor wear. This personalized and efficient operation enhances the overall user experience, making the device more intuitive and reliable. By providing accurate and timely health insights that are tailored to the user's habits, the device encourages regular use and consistent health monitoring, supporting proactive health management.

To further enhance the capability of performing background gas measurements using historic use data, a machine learning model can be employed. This model is trained on the historical usage patterns of the personal care device, allowing it to learn and predict optimal times for conducting background gas measurements. By leveraging machine learning, the device can dynamically adapt to the user's specific habits and environmental conditions, providing a more personalized and accurate assessment. The machine learning model analyzes a wide range of historical data, including the frequency and timing of device use, typical environmental conditions during use, and any variations in ambient gas concentrations. Through this analysis, the model identifies patterns and correlations that are not immediately apparent through manual observation. As a result, the device can anticipate when background gas measurements should be conducted to establish an accurate baseline of ambient gas concentrations. The integration of a machine learning model offers several advantages. First, it enhances measurement accuracy by ensuring that background measurements are tailored to the user's unique usage patterns and environmental conditions. This leads to a more precise differentiation between ambient and user-produced gases, resulting in more reliable health assessments. Second, the use of machine learning optimizes the device's operational efficiency. By predicting when background measurements are most needed, the device can reduce unnecessary measurements, conserving sensor resources and processing power. This contributes to extending battery life and reducing sensor wear, enhancing the device's longevity and performance.

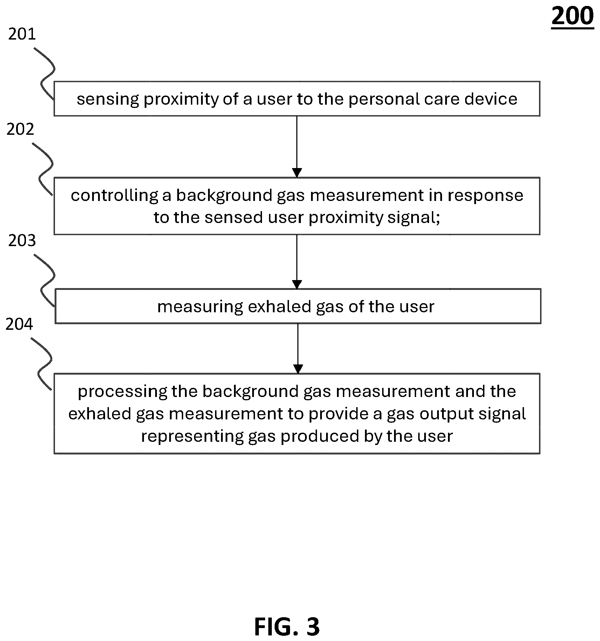

According to an embodiment of the **second** aspect of the invention, and as illustrated in FIG. 3, a method 200 to provide a gas output signal representing gas produced by a user using a personal care device (100) is presented. FIG. 3 shows a method 200 for providing a gas output signal representing gas produced by a user using a personal care device 100. The method 200 involves a series of steps that enable the device to accurately measure and process gas compounds in the user's exhaled breath or air sent out of the lungs of a user, including sensing proximity of a user to the personal care device 201, controlling a background gas measurement in response to the sensed user proximity signal 202, measuring exhaled gas of the user, for example when a care functionality of the personal care device is activated 203, and processing the background gas measurement and the exhaled gas measurement to provide a gas output signal representing gas produced by the user 204. This method can be implemented by the personal care device 100. The second aspect of the invention is closely related to the first aspect, which details the personal care device 100. The description and explanation provided for the first aspect and its embodiment are equally applicable to the second aspect of the invention.

At step 201, the method 200 involves sensing the proximity of a user to the personal care device 100. This step plays a significant role as this action triggers the subsequent operations of the device, ensuring that measurements are taken at appropriate times, thereby enhancing the accuracy of the gas output signal 204. At step 202, the method 200 performs a background gas measurement in response to the sensed user proximity signal 201. This step establishes a baseline of ambient gas concentrations, allowing the device to differentiate between gases present in the environment and those exhaled by the user 203. At step 203, the method 200 measures the exhaled gas of the user, preferably, for example when a care functionality of the personal care device 100 is activated. This step ensures that the device 100 captures the relevant gas compounds produced by the user, which are indicative of various health conditions. At step 204, the method 200 processes the background gas measurement and the exhaled gas measurement to provide a gas output signal representing gas produced by the user. This step involves analyzing the data to generate a reliable signal that reflects the user's health status, free from ambient interference.

To technically realize the processing of background gas measurements and exhaled gas measurements to provide a gas output signal representing gas produced by the user, the following steps and components can be implemented:
**Data Acquisition:** Continuously collect data from the first sensor, capturing both background gas measurements and exhaled gas measurements. Ensure that the data is timestamped and tagged based on proximity signals to distinguish between ambient and user-produced gases.
**Data Segmentation:** Use the proximity detection signals to segment the data into background and exhaled gas measurements. This segmentation is crucial for identifying which portions of the data correspond to ambient conditions and which are related to the user's exhaled breath.
**Optionally Noise Reduction:** Apply noise reduction techniques, such as filtering algorithms, to remove any sensor noise or transient fluctuations that could affect the accuracy of the measurements. This step helps in refining the data quality before further processing.
**Optionally Baseline Correction:** Use the background gas measurements to establish a baseline of ambient gas concentrations. Subtract this baseline from the exhaled gas measurements to correct for any ambient interference, ensuring that the resulting data reflects only the user-produced gases.
**Signal Processing:** Implement advanced signal processing techniques, such as Fourier transforms or wavelet analysis, to analyze the corrected exhaled gas data. These techniques help in identifying specific gas compounds and their concentrations, which are indicative of the user's health status.
**Data Integration:** Integrate the processed data to generate a comprehensive gas output signal. This signal should represent the concentration and composition of gases produced by the user, free from ambient interference.
**Optionally Health Assessment Algorithms:** Use health assessment algorithms to interpret the gas output signal. These algorithms can correlate specific gas compounds with potential health conditions, providing insights into the user's health status.
**Optionally Machine Learning Enhancement:** Optionally, incorporate machine learning models to enhance the accuracy and reliability of the data processing. These models can learn from historical data to improve baseline correction and signal interpretation, adapting to individual user patterns and environmental conditions.

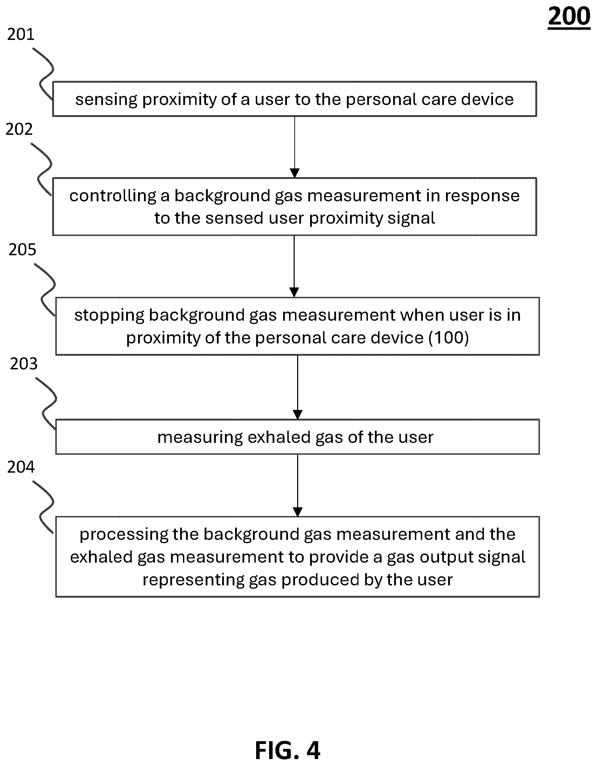

According to an embodiment, the background gas measurement is stopped when the user is in proximity of the personal care device 100. Also illustrated in FIG. 4.

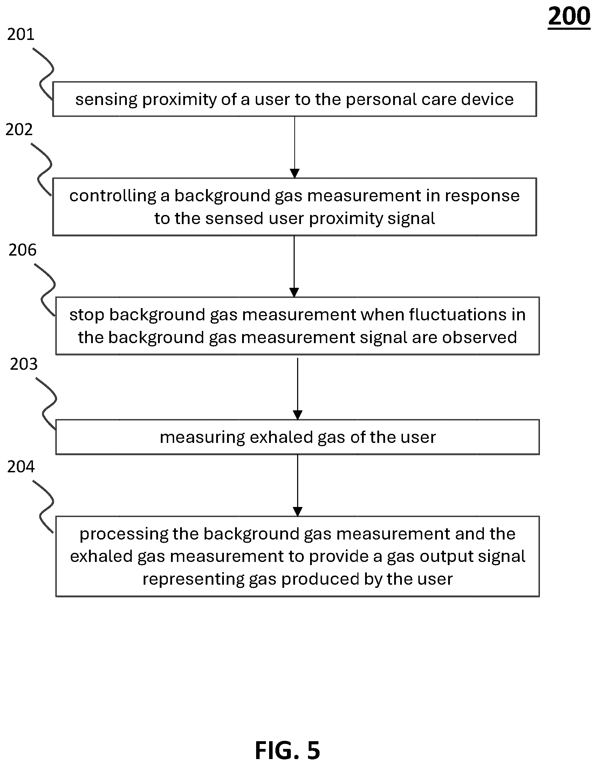

According to an embodiment, the background gas measurement is stopped when fluctuations in the background gas measurement signal are observed (206). Also illustrated in FIG. 5.

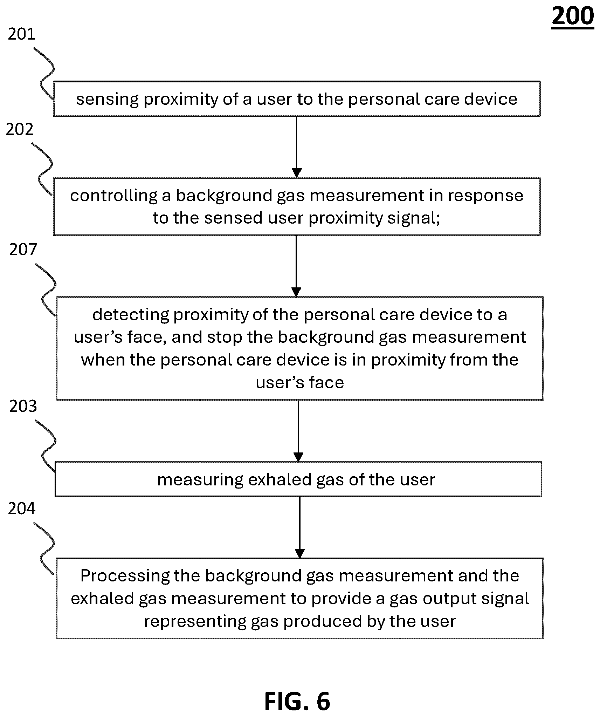

According to an embodiment, the method further comprises detecting proximity of the personal care device 100 to a user's face, and wherein the background gas measurement is stopped when the personal care device 100 is in proximity from the user's face 207. Also illustrated in FIG. 6.

According to an embodiment, the background gas measurement is performed when the user proximity signal indicates that the user is in proximity of the device.

In a **third** aspect of the invention, a computer program product comprising a non-transitory computer-readable medium having stored thereon instructions which, when executed by a processor, cause the processor to perform a method for providing a gas output signal representing gas produced by a user using a personal care device 100, the method comprising the steps 201 to 204: at step 201, the method 200 involves sensing the proximity of a user to the personal care device 100. This step plays a significant role as this action triggers the subsequent operations of the device, ensuring that measurements are taken at appropriate times, thereby enhancing the accuracy of the gas output signal 204. At step 202, method 200 controls a background gas measurement in response to the sensed user proximity signal 201. This step establishes a baseline of ambient gas concentrations, allowing the device to differentiate between gases present in the environment and those exhaled by the user 203. At step 203, the method 200 measures the exhaled gas of the user, preferably when a care functionality of the personal care device 100 is activated. This step ensures that the device 100 captures the relevant gas compounds produced by the user, which are indicative of various health conditions. At step 204, method 200 processes the background gas measurement and the exhaled gas measurement to provide a gas output signal representing gas produced by the user. This step involves analyzing the data to generate a reliable signal that reflects the user's health status, free from ambient interference.

Throughout the description, the term "exhaled gas" of the user refers to gas or air that is sent out of the lung of the user. This may be gas or air that is present in the oral cavity or gas or air that is exhaled outside of the body of the user, or any gas that is produced by the body of the user.

In general, measurement reaction time is a combination of the gas sensor response time and the diffusion rate of the specific gas compound. For fast reacting time: the embodiments can be used, for which background gas concentration is measured in the short time interval between picking up the device (or being close to the device) and the device being close to the user's face. For slow reaction time: it makes more sense to use embodiments, for which background measurement is done when the user is not close, and background measurement is stopped once the device is picked/user is close to the device. As this allows for longer measurement intervals.

## Claims

1. A personal care device (100) comprising:
a first sensor (101) for measuring gas compounds;
a means (102) for detecting proximity of a user to the personal care device, the means providing a proximity output signal;
a processor (103) configured for:
controlling the first sensor (101) for performing a background gas measurement, in response to the proximity output signal; thereafter
controlling the first sensor (101) for performing an exhaled gas measurement;
processing the background gas measurement and the exhaled gas measurement to provide a gas output signal representing gas produced by the user.

2. The personal care device (100) according to claim 1, wherein the means (102) is a touch-sensitive proximity sensor.

3. The personal care device (100) according to claim 1, wherein the means (102) is an IMU.

4. The personal care device (100) according to any of the preceding claims, wherein the processor (103) is further configured to control the first sensor (101) such that the background gas measurement is stopped when the personal care device (100) is switched on.

5. The personal care device (100) according to any of the preceding claims, wherein the processor (103) is further configured to control the first sensor (101) such that the background gas measurement is stopped when fluctuations in the first sensor (101) output signal are observed.

6. The personal care device (100) according to any of the preceding claims, further comprising a second sensor (104) for detecting proximity of the personal care device (100) to a user's face, and wherein the processor (103) is further configured to control the first sensor (101) such that the background gas measurement is stopped when the personal care device (100) is in proximity of the user's face within a predetermined limit.

7. The personal care device (100) according to any of the preceding claims, and wherein processing the background gas measurement and the exhaled gas measurement to provide a gas output signal comprises discarding or selecting a portion of the background gas measurement.

8. The personal care device (100) according to any of the preceding claims, wherein the background gas measurement is performed when the proximity signal indicates that the user is in proximity of the device (100).

9. The personal care device (100) according to any of claims 1 to 8, wherein the background gas measurement is performed as long as the proximity signal indicates that the user is not in proximity of the device.

10. The personal care device (100) according to any of the preceding claims, wherein timing of performing the background gas measurement is determined using historic use data of the personal care device (100).

11. A method (200) for providing a gas output signal representing gas produced by a user using a personal care device (100), comprising:
sensing (201) proximity of a user to the personal care device (100);
controlling (202) a background gas measurement in response to the sensed user proximity signal;
measuring (203) exhaled gas of the user resulting in a exhaled gas measurement;
processing (204) the background gas measurement and the exhaled gas measurement to provide a gas output signal representing gas produced by the user.

12. The method (200) according to claim 11, wherein the background gas measurement is stopped (205) when the user is in proximity of the personal care device (100).

13. The method (200) according to any of claims 11 to 12, wherein the background gas measurement is stopped when fluctuations in the background gas measurement signal are observed (206).

14. The method (200) according to any of claims 11 to 13, further comprising detecting proximity of the personal care device (100) to a user's face, and wherein the background gas measurement is stopped when the personal care device (100) is in proximity from the user's face (207).

15. The method (200) according to any of claims 11 to 14, wherein the background gas measurement is performed when the user proximity signal indicates that the user is in proximity of the device.
